# EUROPÄISCHE PATENTSCHRIFT

(11) **EP 3 784 310 B1**
(45) Veröffentlichungstag und Bekanntmachung des Hinweises auf die Patenterteilung: **02.10.2024**
(21) Anmeldenummer: 19721575.9
(22) Anmeldetag: 26.04.2019
(51) Int. Cl.: A61M 1/36, A61M 1/16

(54) **VORRICHTUNG ZUM HALTEN EINES MEDIZINISCHEN EINMALARTIKELS ZUR BLUTBEHANDLUNG**
APPARATUS FOR HOLDING A DISPOSABLE MEDICAL ITEM FOR TREATING BLOOD
DISPOSITIF DE MAINTIEN D'UN ARTICLE MÉDICAL À USAGE UNIQUE POUR LE TRAITEMENT DU SANG

(30) Priorität: 27.04.2018 DE 102018206633
(43) Veröffentlichungstag der Anmeldung: 03.03.2021
(73) Patentinhaber: Fresenius Medical Care Deutschland GmbH, 61352 Bad Homburg (DE)
(72) Erfinder: SCHRÖRS, Alexander, 60389 Frankfurt (DE); CZERWONKA, Sven Marten, 60437 Frankfurt (DE); PARTENFELDER, Robin, 61440 Oberursel (DE); MOSDZINSKI, Alexander, 85591 Vaterstetten (DE); HAHN, Lena Maria, 53119 Bonn (DE); WALDE, Jan-Willem, 35440 Linden (DE)
(74) Vertreter: Ricker, Mathias
(86) Internationale Anmeldenummer: PCT/EP2019/060695
(87) Internationale Veröffentlichungsnummer: WO 2019/207082

(56) Entgegenhaltungen:
- DE-A1- 102005 021 305
- US-A- 5 770 064
- US-A1- 2009 101 576
- US-B2- 9 895 700

## Beschreibung

Die vorliegende Erfindung betrifft eine Vorrichtung zum Halten eines medizinischen Einmalartikels zur Blutbehandlung und ein System mit einer solchen Vorrichtung.

Zur Behandlung von Blut eines Patienten sind Blutbehandlungsvorrichtungen mit einem Einmalartikel bekannt, etwa Dialysevorrichtungen mit einem Dialysator. Der Dialysator weist eine semipermeable Membran auf, die den Dialysator in zwei Bereiche trennt, wobei durch einen der Bereiche Blut und durch den anderen der Bereiche ein Dialysat geleitet wird, so dass kleine Moleküle wie etwa Wasser, Elektrolyte und harnpflichtige Substanzen aus dem Blut durch die Membran in das Dialysat treten und abtransportiert werden können.

Andere medizinische Einmalartikel zur Behandlung von Blut sind beispielsweise Plasmafilter oder Adsorber. Plasmafilter weisen eine Membran auf, mittels der durch Anlegen eines Transmembrandrucks Ultrafiltrat, d.h. Blutplasma, aus an der Membran entlangströmendem Blut eines Patienten abgetrennt wird.

Ein Adsorber erlaubt es, pathogene Stoffe aus Vollblut oder Blutplasma eines Patienten zu entfernen. Adsorber werden beispielsweise beim sog. Plasmaphereseverfahren, in dem das Blut in Blutplasma und korpuskulare Bestandteile, etwa Zellen, aufgetrennt wird. Das dabei abgetrennte Blutplasma wird in bevorzugter Weise über den Adsorber gereinigt und zum Patienten zurückgeführt.

Um den Betrieb solcher Einmalartikel zu vereinfachen, können Einmalartikel üblicherweise mithilfe einer Halterung an der Blutbehandlungsvorrichtung angebracht werden. Dabei kann es nötig oder zumindest gewünscht sein, den Einmalartikel neu auszurichten, um einen platzsparenden und wenig hinderlichen Verlauf von Schläuchen, welche den Einmalartikel mit der Blutbehandlungsvorrichtung verbinden, zu bewirken. Darüber hinaus kann die Neuausrichtung des Einmalartikels auch notwendig oder zumindest gewünscht sein, um bei der Befüllung des Einmalartikels zumindest mit Blut, gegebenenfalls auch mit einer weiteren Flüssigkeit wie Dialysat, Substituat und/oder einer Spülflüssigkeit, den Einschluss von Luft im Einmalartikel zu verhindern oder zumindest zu verringern.

Die DE 10 2005 021 305 A1 offenbart eine Reaktoreinheit mit einer ersten Kammer und einer zweiten Kammer, wobei die erste Kammer durch den Innenraum eines Gehäuses gebildet wird und wobei die zweite Kammer durch den Innenraum mehrerer in dem Gehäuse angeordneter Hohlfasern gebildet wird. Die Hohlfasern sind in dem Gehäuse derart angeordnet, dass ihre auf die Querschnittsfläche der ersten Kammer bezogene Dichte in wenigstens einem Bereich der ersten Kammer 10 Fasern/mm² nicht übersteigt. Gemäß einer weiteren Ausgestaltung ist vorgesehen, dass in der Reaktoreinheit zwei Vergussmassen angeordnet sind, in denen ein Abschnitt der Hohlfasern eingebettet ist und zwischen denen sich ein weiterer Abschnitt der Hohlfasern erstreckt, wobei die Länge wenigstens einiger oder aller Hohlfasern mindestens 0,5% über dem Abstand der Vergussmassen liegt.

Die US 9 895 700 B2 offenbart Systeme und Verfahren zur Verbesserung der Durchflussrate des Plasmas, das während eines Bluttrennverfahrens aus einer Bluttrennkammer entfernt wird, wobei das System eine Bluttrennkammer, in der Plasma von zellulären Blutbestandteilen getrennt wird, und eine Auslassleitung zum Entfernen des abgetrennten Plasmas aus der Bluttrennkammer umfasst, wobei eine primäre optische Sensoreinheit mit der Bluttrennkammer verbunden ist, um das Innere der Bluttrennkammer direkt zu überwachen, eine sekundäre optische Sensoreinheit mit der Auslassleitung verbunden ist, um das abgetrennte Plasma in der Auslassleitung zu überwachen, und jede optische Sensoreinheit das Vorhandensein von zellulären Bestandteilen im abgetrennten Plasma überprüft. Wenn die primäre optische Sensoreinheit einen solchen Zustand feststellt, die sekundäre optische Sensoreinheit jedoch nicht, kann ein Steuergerät Schritte unternehmen, um den Konflikt zu lösen und das Verfahren fortzusetzen.

Die US 5 770 064 A offenbart einen kombinierten Halter und Verbinder für eine Dialysemaschine zum Betreiben und Überwachen von Dialysevorrichtungen, einschließlich eines Halters, der um eine Halteachse schwenkbar ist und sich von der Dialysemaschine zur Handhabung von Flüssigkeit erstreckt, die innerhalb der Dialysemaschine verarbeitet werden soll, wobei der Halter einen Verbinder umfasst, um den Halter mit einem Flüssigkeitskoppler zu verbinden, so dass die Flüssigkeit zwischen dem Flüssigkeitskoppler und der Dialysemaschine fließen kann, wobei die Verbindungsachse des Verbinders quer zur Halterachse des Halters verläuft.

Es ist eine Aufgabe der Erfindung, die Ausrichtung eines medizinischen Einmalartikels zur Blutbehandlung zu verbessern, insbesondere zuverlässiger, sicherer und/oder komfortabler zu machen.

Diese Aufgabe wird gelöst durch eine Vorrichtung zum Halten eines medizinischen Einmalartikels zur Blutbehandlung und ein System mit einer solchen Vorrichtung gemäß den unabhängigen Ansprüchen.

Eine erfindungsgemäße Vorrichtung zum Halten eines medizinischen Einmalartikels zur Blutbehandlung weist eine Lagereinrichtung, die dazu eingerichtet ist, den Einmalartikel drehbar zu lagern, und eine Antriebseinrichtung auf, die dazu eingerichtet ist, den drehbar gelagerten Einmalartikel um eine Längsachse, die im Wesentlichen in einer Richtung verläuft, in der im Betrieb des Einmalartikels Blut durch den Einmalartikel fließt, zu drehen. Zusätzlich weist die Vorrichtung eine Sensoreinrichtung auf, die dazu eingerichtet ist, eine Drehung des drehbar gelagerten Einmalartikels um wenigstens die Längsachse zu erfassen und entsprechende Sensordaten auszugeben. Eine Steuerungseinrichtung ist ferner dazu eingerichtet, die Antriebseinrichtung zu steuern und die von der Sensoreinrichtung ausgegebenen Sensordaten zu verarbeiten.

Ein erfindungsgemäßes System zur Blutbehandlung weist eine erfindungsgemäße Vorrichtung, einen Einmalartikel, der von der erfindungsgemäßen Vorrichtung gehalten wird, und eine Blutbehandlungsvorrichtung auf, die mit dem Einmalartikel verbunden und dazu eingerichtet ist, zumindest Blut zur Behandlung des Bluts durch den Einmalartikel zu leiten.

Bei einem Verfahren zum Betrieb eines Einmalartikels, das nicht Teil der Erfindung ist, wird der Einmalartikel drehbar gelagert und mittels einer Antriebseinrichtung um eine Längsachse, die im Wesentlichen in einer Richtung verläuft, in der im Betrieb des Einmalartikels Blut durch den Einmalartikel fließt, gedreht und/oder von einer Sensoreinrichtung eine Drehung des drehbar gelagerten Einmalartikels um wenigstens eine Drehachse, insbesondere um die Längsachse und/oder um eine zur Längsachse senkrecht verlaufende Querachse, erfasst. Dabei werden entsprechende Sensordaten ausgegeben.

Ein Aspekt der Erfindung basiert auf dem Ansatz, eine notwendige und/oder gewünschte Ausrichtung des Einmalartikels durch eine automatische, d.h. mittels einer Antriebseinrichtung bewirkte, Drehung des Einmalartikels um eine Längsachse zu erreichen und die Ausrichtung des Einmalartikels, insbesondere die mittels der Antriebseinrichtung bewirkte Drehung des Einmalartikels um wenigstens die Längsachse, mittels einer Sensoreinrichtung zu überwachen. Dies ermöglicht eine präzise und zuverlässige Ausrichtung des Einmalartikels bzw. deren Überwachung und somit einen sicheren und komfortablen Betrieb des Einmalartikels.

Die Vorrichtung zum Halten des Einmalartikels weist vorzugsweise eine Lagereinrichtung auf, die beispielsweise als Greifeinrichtung ausgebildet sein kann und die den Einmalartikel zumindest teilweise umgreift. Die Lagereinrichtung weist vorzugsweise eine dem Einmalartikel zugewandte Innenseite auf, an welcher Lagerelemente vorgesehen sein können, die zur drehbaren Lagerung des Einmalartikels um die Längsachse eingerichtet sind. Beispielsweise kann die Lagereinrichtung zumindest drei Räder und/oder Rollen aufweisen, die beim Halten des Einmalartikels mit dem Einmalartikel in Kontakt stehen. Über die Räder kann der Einmalartikel drehbar um die Längsachse gelagert sein, wobei zumindest eines der Räder und/oder eine der Rollen mit der vorzugsweise als Elektromotor, welcher insbesondere derart ausgeführt ist, dass die Drehposition der Motorwelle kontrollierbar und/oder bestimmbar ist, wie z.B. als Servomotor oder Schrittmotor, ausgebildeten Antriebseinrichtung verbunden sein kann, um eine automatische Drehung des Einmalartikels um die Längsachse bewirken.

Die Lagereinrichtung kann dabei auch weitere Lagerelemente aufweisen, welche die Drehung des Einmalartikels um die Querachse ermöglichen. Beispielsweise kann die Greifeinrichtung um die Querachse gedreht werden. Dabei ist vorzugsweise auch zumindest eines der weiteren Lagerelemente mit der Antriebseinrichtung verbunden, um eine automatische Drehung des Einmalartikels um die Querachse zu bewirken.

Dadurch kann der Einmalartikel, insbesondere mittels Drehung um die Längsachse, z.B. derart ausgerichtet werden, dass Schläuche einer Dialysevorrichtung komfortabel an entsprechende Kupplungen des Einmalartikels angeschlossen werden können. Anschließend kann der Einmalartikel automatisch in eine Stellung verbracht werden, in der z.B. die Kupplungen und/oder Schläuche einen Benutzer, etwa behandelndes Personal oder einen Patienten, nicht behindern, wodurch das Risiko einer Beschädigung der Kupplungen und/oder der Schläuche verringert wird.

Zusätzlich kann mittels der Sensoreinrichtung zudem die automatische, alternativ aber auch eine manuelle, Drehung des Einmalartikels um wenigstens die Längsachse überwacht werden, um Fehlausrichtungen, insbesondere beim initialen Befüllen des Einmalartikels mit Blut und/oder Dialysat und/oder beim Spülen des Einmalartikels mit einer Spülflüssigkeit zu detektieren und gegebenenfalls eine entsprechende Benachrichtigung über eine Ausgabeeinrichtung, die Teil der Vorrichtung zum Halten des Einmalartikels oder der Dialysevorrichtung sein kann, auszugeben. Dadurch kann beispielsweise verhindert werden, dass Luft im Einmalartikel beim Befüllen eingeschlossen wird und/oder ein mit dem Einmalartikel verbundener Schlauch, etwa durch die Blutbehandlungsvorrichtung, abgeklemmt oder zumindest geknickt wird.

Insgesamt erlaubt die Erfindung eine verbesserte, insbesondere eine definierte Ausrichtung eines Einmalartikels, insbesondere die Ausrichtung des Einmalartikels bzw. deren Überwachung zuverlässiger, sicherer und/oder komfortabler zu machen.

Die Sensoreinrichtung weist einen optischen Sensor auf, der dazu eingerichtet ist, eine am Einmalartikel befindliche Markierung, insbesondere einen Strichcode, bei und/oder nach der Drehung des Einmalartikels um die Längsachse zu erfassen und entsprechende erste Sensordaten auszugeben. Die Steuerungseinrichtung ist dazu eingerichtet, anhand der vom optischen Sensor ausgegebenen ersten Sensordaten eine Information betreffend die Drehung und/oder die Drehstellung des Einmalartikels bezüglich der Längsachse zu ermitteln. Dadurch ist die Ausrichtung des Einmalartikels, insbesondere von Kupplungen des Einmalartikels und/oder damit verbundenen Schläuchen, beispielsweise relativ zur Blutbehandlungsvorrichtung, bei oder nach einer manuellen oder automatischen Drehung des Einmalartikels um die Längsachse besonders präzise und zuverlässig bestimmbar.

Der optische Sensor kann etwa als Kamera oder Zeilensensor ausgebildet sein, die bzw. der dazu eingerichtet ist, ein vorgegebenes Merkmal, beispielsweise ein auf dem Einmalartikel angebrachtes Label mit einem Strichcode oder eine bauartbedingte Form des Einmalartikels, zu erfassen und entsprechende Bilddaten zu erzeugen. Die Steuerungseinrichtung ist dabei vorzugsweise dazu eingerichtet, die Bilddaten durch einen Algorithmus zur Bilderkennung zu analysieren, dabei die Lage des Merkmals auf dem Einmalartikel zu erkennen und daraus auf die Drehung und/oder die Drehstellung zu schließen.

Bei einer nicht unter den Anspruchsgegenstand fallenden Ausführungsform kann die Sensoreinrichtung als Magnetsensor, etwa als Spuleneinrichtung, ausgebildet und dazu eingerichtet sein, eine am Einmalartikel befindliche magnetische Markierung bei und/oder nach der Drehung des Einmalartikels um wenigstens die Längsachse zu erfassen und entsprechende erste Sensordaten auszugeben. Dies kann insbesondere vorteilhaft sein, wenn für den Betrieb eines optischen Sensors nicht ausreichend Licht zur Verfügung steht.

In einer weiteren bevorzugten Ausführungsform weist die Sensoreinrichtung einen Kraftsensor, insbesondere einen Drehmomentsensor, auf, der dazu eingerichtet ist, eine auf den Einmalartikel bei der Drehung um wenigstens die Längsachse wirkende Kraft zu erfassen und entsprechende zweite Sensordaten auszugeben. Die Steuerungseinrichtung ist dabei vorzugsweise dazu eingerichtet, anhand der vom Kraftsensor ausgegebenen zweiten Sensordaten einen Betriebszustand des Einmalartikels zu ermitteln. Dadurch kann unmittelbar und zuverlässig festgestellt werden, ob der Betrieb des Einmalartikels fehlerfrei, insbesondere ohne Beschädigungsrisiko für den Einmalartikel, möglich ist.

Der Betriebszustand kann beispielsweise die Situation, in der sich der Einmalartikel befindet und/oder betrieben wird, charakterisieren. Der Betriebszustand kann sich etwa auf einen Füllzustand des Einmalartikels mit einer Flüssigkeit beziehen, d.h. der Betriebszustand kann z.B. eine Information liefern, ob der Einmalartikel leer, teilweise oder vollständig mit einer Flüssigkeit, insbesondere Blut und/oder beispielsweise einem Dialysat oder einem Substituat, befüllt ist. Alternativ oder zusätzlich kann sich der Betriebszustand auf eine Ausrichtung und/oder Position des Einmalartikels, insbesondere relativ zur Blutbehandlungsvorrichtung, beziehen, d.h. der Betriebszustand kann z.B. eine Information liefern, ob der Einmalartikel senkrecht oder waagerecht ausgerichtet ist oder ob der Einmalartikel, insbesondere Kupplungen des Einmalartikels, mit einem Hindernis in Kontakt steht.

Anhand des ermittelten Betriebszustands kann unter anderem vorteilhaft verhindert werden, dass bei einer Ausrichtung des Einmalartikels Kräfte auf den Einmalartikel einwirken, welche den Einmalartikel beschädigen, wenn der Einmalartikel beispielsweise derart gedreht wird, dass er in Kontakt mit der Blutbehandlungsvorrichtung steht.

Der Kraftsensor, beispielsweise ein Drehmomentsensor, kann insbesondere mit der Lagereinrichtung und/oder der Antriebseinrichtung verbunden oder gegebenenfalls in der Lagereinrichtung bzw. der Antriebseinrichtung verbaut sein. Der Kraftsensor ist vorzugsweise dazu eingerichtet, Widerstände bei der Drehung des Einmalartikels um wenigstens die Längsachse zu detektieren. Dadurch können die zweiten Sensordaten besonders zuverlässig erzeugt bzw. der Betriebszustand besonders differenziert ermittelt werden.

In einer weiteren bevorzugten Ausführungsform ist die Steuerungseinrichtung dazu eingerichtet, anhand der vom Kraftsensor ausgegebenen zweiten Sensordaten ein Maß für das Gewicht des Einmalartikels, insbesondere dessen Leergewicht, zu ermitteln. Vorzugsweise ist die Steuerungseinrichtung dazu eingerichtet, anhand der zweiten Sensordaten, insbesondere anhand des Maßes für das Leergewicht, einen Typ des Einmalartikels, etwa dessen Größe, zu ermitteln, und diese Information gegebenenfalls an einen Benutzer auszugeben. Beispielsweise kann die Steuerungseinrichtung anhand der zweiten Sensordaten bei einer Drehung des Einmalartikels um wenigstens eine der Drehachsen mittels der Antriebseinrichtung die auf den Einmalartikel wirkende Beschleunigung ermitteln und daraus ein Maß für das Gewicht ableiten. Alternativ oder zusätzlich kann in einer vorgegebenen Drehstellung des Einmalartikels, in der beispielsweise die Längsachse waagrecht ausgerichtet ist, aus den zweiten Sensordaten die Kraft, die auf die Lagereinrichtung einwirkt, ermittelt werden und daraus die Gewichtskraft bzw. ein Maß für das Gewicht des Einmalartikels ableiten. Dadurch kann zuverlässig überprüft werden, ob für eine beabsichtigte Behandlung, etwa eines Kindes, der passende Einmalartikel von der Vorrichtung gehalten wird.

Gegebenenfalls kann mittels der Steuerungseinrichtung anhand des Maßes für das Gewicht des Einmalartikels auch der Füllstand einer Flüssigkeit im Einmalartikel abgeleitet, insbesondere eine nicht vollständige Füllung des Einmalartikels erkannt, werden. Dadurch kann, insbesondere automatisch, überprüft werden, ob der Einmalartikel einsatzbereit, z.B. vollständig mit Dialysat oder Substituat befüllt, ist und somit auch sichergestellt werden, dass ein Patient eine korrekte Behandlung erhält.

In einer weiteren bevorzugten Ausführungsform ist die Steuerungseinrichtung dazu eingerichtet, die Antriebseinrichtung anhand der von der Sensoreinrichtung ausgegebenen Sensordaten und/oder des ermittelten Betriebszustands und/oder Maßes für das Gewicht des Einmalartikels zu steuern. Dadurch kann der Komfort für einen Benutzer und/oder die Betriebssicherheit weiter erhöht werden.

Beispielsweise kann bei der Erkennung eines Widerstands eine mittels der Antriebseinrichtung ausgeführte Drehung gestoppt werden, etwa bei Feststellen, dass ein vorgegebener Kraftschwellenwert von der auf den Einmalartikel wirkenden Kraft erreicht oder überschritten wird. Alternativ oder zusätzlich kann der Einmalartikel in eine für einen Behandlungsschritt notwendige oder vorteilhafte Drehstellung gedreht werden, nachdem die aktuelle Drehstellung erkannt wurde.

In einer weiteren bevorzugten Ausführungsform ist die Steuerungseinrichtung dazu eingerichtet, die Antriebseinrichtung derart zu steuern, dass der Einmalartikel abwechselnd vor und zurück um wenigstens die Längsachse gedreht wird. Vorzugsweise ist die Steuerungseinrichtung dazu eingerichtet, die Antriebseinrichtung derart zu steuern, dass sich die Drehrichtung der Drehung um wenigstens die Längsachse abrupt und/oder mit hoher Frequenz, etwa mit 1 Hz oder schneller, bevorzugt mit 5 Hz oder schneller, insbesondere mit 10 Hz oder schneller, ändert. Insbesondere kann die Steuerungseinrichtung dabei dazu eingerichtet sein, die Antriebseinrichtung derart zu steuern, dass eine Schüttelbewegung oder Vibration des Einmalartikels erzeugt wird. Durch eine solche Schüttelbewegung oder Vibration kann eine sich im Einmalartikel befindliche Flüssigkeit, etwa Blut und/oder eine Dialyseflüssigkeit oder ein Substituat, das bzw. die den Einmalartikel durchfließt, von Luftblasen befreit werden.

Die Steuerungseinrichtung kann z.B. dazu eingerichtet sein, das Vor- und Zurückdrehen des Einmalartikels auf Grundlage eines extern erzeugten Signals zu veranlassen, beispielsweise auf Grundlage eines bei der Detektion von Luftblasen in einer den Einmalartikel durchfließenden Flüssigkeit automatisch erzeugten Detektionssignals. Alternativ kann es sich bei dem extern erzeugten Signal aber auch um ein durch einen Benutzer manuell erzeugtes Eingabesignal handeln. So kann ein Benutzer etwa nach einer Benachrichtigung, dass Luftblasen detektiert wurden, oder zur Entlüftung beim Befüllen des Einmalartikels mit einer Flüssigkeit, die Vibration des Einmalartikels manuell veranlassen. Die Vibration kann alternativ oder zusätzlich aber auch zyklisch ausgeführt werden. Dazu ist die Steuerungseinrichtung vorzugsweise dazu eingerichtet, das Vor- und Zurückdrehen des Einmalartikels mittels der Antriebseinrichtung in vorgegebenen Zeitintervallen oder in Abhängigkeit der Menge des durch den Einmalartikel geflossenen Blutes zu veranlassen.

In einer weiteren bevorzugten Ausführungsform ist der medizinische Einmalartikel zur Blutbehandlung als Dialysator, Plasmafilter oder Adsorber ausgebildet. Insbesondere kann die Lagereinrichtung derart konfiguriert, insbesondere geformt, sein, dass ein Dialysator, Plasmafilter oder Adsorber zumindest teilweise umschlossen wird und/oder mittels eines Rast- und/oder Clipmechanismus an einer drehbar gelagerten Komponente der Lagereinrichtung fixierbar ist. Dialysatoren, Plasmafilter oder Adsorber können dadurch besonders zuverlässig betrieben werden.

In einer weiteren bevorzugten Ausführungsform weist der Einmalartikel eine Markierung, insbesondere einen Strichcode, auf, wobei die Markierung vorzugsweise entlang eines Umfangs des Einmalartikels verläuft. Dadurch kann mittels der Sensoreinrichtung, insbesondere eines optischen Sensors wie etwa einer Kamera oder eines magnetischen Sensors wie etwa einer Spuleneinrichtung, unkompliziert und zuverlässig eine Drehung und/oder Drehstellung des Einmalartikels bezüglich der Längsachse ermittelt werden.

In einer weiteren bevorzugten Ausführungsform weist der Einmalartikel wenigstens zwei Öffnungen auf, durch die im Betrieb des Einmalartikels eine Flüssigkeit in den Einmalartikel eintreten und/oder aus dem Einmalartikel austreten kann. Die Steuerungseinrichtung ist dabei vorzugsweise dazu eingerichtet, die Antriebseinrichtung derart zu steuern, dass der Einmalartikel in eine Befüllstellung gedreht wird, in der zumindest eine der Öffnungen im Wesentlichen bis zum vollständigen Befüllen des Einmalartikels mit der Flüssigkeit oberhalb des Flüssigkeitspegels der Flüssigkeit liegt, so dass beim Befüllen des Einmalartikels mit der Flüssigkeit Luft durch die zumindest eine der Öffnungen aus dem Einmalartikel entweichen kann. Dadurch kann ein zuverlässiger Betrieb des Einmalartikels sichergestellt werden.

Bei der Flüssigkeit kann es sich insbesondere um eine Kochsalzlösung, ein Dialysat oder Substituat handeln, das zur Behandlung eines Patienten bzw. zur Reinigung durch den Einmalartikel geleitet wird.

Die Steuerung der Antriebseinrichtung durch die Steuerungseinrichtung kann insbesondere auf Grundlage einer ermittelten Drehstellung des Einmalartikels, etwa anhand der sensorisch erfassten Markierung auf dem Einmalartikel, ausgeführt werden. Dadurch kann der Einmalartikel beispielsweise derart ausgerichtet werden, dass die Längsachse im Wesentlichen senkrecht steht oder um einen vorgegebenen Winkel gegenüber einer senkrechten Ausrichtung verkippt ist, da sich die Öffnungen zum Befüllen des Einmalartikels in der Regel an zwei in Richtung der Längsachse einander gegenüberliegenden Enden des Einmalartikels befinden. Alternativ oder zusätzlich kann die Steuerungseinrichtung dazu eingerichtet sein, die Antriebseinrichtung derart zu steuern, dass der um einen vorgegebenen Winkel gegenüber einer senkrechten Ausrichtung verkippte Einmalartikel um die Längsachse gedreht wird, bis eine senkrecht zur Längsachse ausgerichtete Öffnung, beispielsweise eine Kupplung des Einmalartikels zum Anschluss an die Blutbehandlungsvorrichtung mittels eines Schlauchs, auf einer Oberseite des Einmalartikels liegt.

In einer weiteren bevorzugten Ausführungsform weist die Blutbehandlungsvorrichtung wenigstens einen Detektor auf, der dazu eingerichtet ist, in einer durch den Einmalartikel geleiteten Flüssigkeit vor dem Eintritt der Flüssigkeit in den Einmalartikel und/oder nach dem Austritt der Flüssigkeit aus dem Einmalartikel Luftblasen zu detektieren und entsprechende Detektorsignale auszugeben. Dabei ist die Steuerungseinrichtung vorzugsweise dazu eingerichtet, basierend auf den Detektorsignalen die Antriebseinrichtung derart zu steuern, dass der Einmalartikel abwechselnd vor und zurück um wenigstens eine Drehachse gedreht wird, insbesondere bis keine Luftblasen mehr detektiert werden. Durch die, insbesondere schnelle und/oder abrupte, Änderung der Drehrichtung des Einmalartikels, beispielsweise mit 1 Hz oder schneller, bevorzugt mit 5 Hz oder schneller, insbesondere mit 10 Hz oder schneller, können Luftblasen aus der Flüssigkeit entfernt werden. Dadurch wird ein zuverlässiger Betrieb des Systems zur Blutbehandlung ermöglicht.

Der Detektor kann insbesondere als Blutleckdetektor zur Detektion von Blut in einer Ableitung für Flüssigkeit aus dem Einmalartikel, z.B. Dialysat oder Filtrat aus dem Dialysator, ausgebildet sein, mit dessen Hilfe aber auch Luftblasen detektiert werden können. Alternativ oder zusätzlich kann der Detektor als Luftblasendetektor zur Detektion von Luftblasen in einer Zu- und/oder Ableitung für Blut in den bzw. aus dem Einmalartikel ausgebildet sein.

In einer weiteren bevorzugten Ausführungsform wird der Einmalartikel zu Beginn des Betriebs, insbesondere zum Spülen mit einer Spülflüssigkeit, mittels der Antriebseinrichtung in eine Befüllstellung gedreht, in der zumindest eine von wenigstens zwei Öffnungen des Einmalartikels, über die im Betrieb des Einmalartikels eine Flüssigkeit in den Einmalartikel eintreten und/oder aus dem Einmalartikel austreten kann, bis zum im Wesentlichen vollständigen Befüllen des Einmalartikels mit der Flüssigkeit oberhalb des Flüssigkeitspegels der Flüssigkeit im Einmalartikel liegt, so dass beim Befüllen des Einmalartikels mit einer Flüssigkeit durch die zumindest eine der Öffnungen Luft aus dem Einmalartikel entweichen kann. Vorzugsweise wird der in der Befüllstellung gehaltene Einmalartikel dann mit einer Flüssigkeit befüllt und um die Querachse um 180° gedreht. Dadurch kann sichergestellt werden, dass die Flüssigkeit den Einmalartikel im Inneren des Einmalartikels vollständig benetzt. Beispielsweise kann eine Reinigung so besonders gründlich durchgeführt werden.

Beim Verbringen des Einmalartikels in die Befüllstellung und/oder beim Drehen um die Querachse in eine Entlüftungsstellung, beispielsweise durch Drehung um ca. 180°, kann die Drehung bzw. Drehstellung des Einmalartikels von der Sensoreinrichtung, insbesondere mittels eines Kraftsensors, überwacht werden, so dass eine Kollision des Einmalartikels und/oder einer mit dem Einmalartikel verbundenen Komponente des Systems zur Blutbehandlung, etwa einer Kupplung des Einmalartikels oder eines Schlauchs zur Verbindung des Einmalartikels mit der Blutbehandlungsvorrichtung, unmittelbar festgestellt und ein Weiterdrehen des Einmalartikels verhindert werden kann. Somit können Beschädigungen des Einmalartikels und/oder anderer Komponenten des Systems zur Blutbehandlung effektiv vermieden werden.

In einer weiteren bevorzugten Ausführungsform wird der in der Befüllstellung gehaltene Einmalartikel während des Befüllens mit der Flüssigkeit abwechselnd vor und zurück um wenigstens die Längsachse gedreht. Dadurch können Lufteinschlüsse im Einmalartikel zuverlässig vermieden oder zumindest reduziert werden.

In einer weiteren bevorzugten Ausführungsform wird der Einmalartikel in vorgegebenen Intervallen abwechselnd vor und zurück um wenigstens die Längsachse gedreht. Bei den vorgegebenen Intervallen kann es sich beispielsweise um zeitliche Intervalle oder durch den Einmalartikel geflossene Blutvolumen handeln, so dass der Einmalartikel in festen Zeitabständen oder wenn eine bestimmte Menge an Blut gereinigt wurde, vor und zurück gedreht wird. Dadurch wird ein sicherer und unterbrechungsfreier Betrieb des Einmalartikels ermöglicht.

Weitere Vorteile, Merkmale und Anwendungsmöglichkeiten der vorliegenden Erfindung ergeben sich aus der nachfolgenden Beschreibung in Zusammenhang mit den Figuren. Es zeigen:
- Fig. 1: ein Beispiel eines Systems zur Dialyse;
- Fig. 2: ein Beispiel einer Lagereinrichtung; und
- Fig. 3: ein Beispiel eines Dialysators.

Auch wenn sich die in den Figuren dargestellten und nachfolgend beschriebenen Ausführungsbeispiele eines Systems, einer Lagereinrichtung und eines als Dialysator ausgebildeten Einmalartikels auf Anwendungen der Erfindung bei der Dialyse beziehen, gelten die folgenden Ausführungen auch für andere Einmalartikel, wie etwa Plasmafilter oder Adsorber, und entsprechende Systeme bzw. Vorrichtungen zur Anwendung bei anderen Blutbehandlungsverfahren entsprechend.

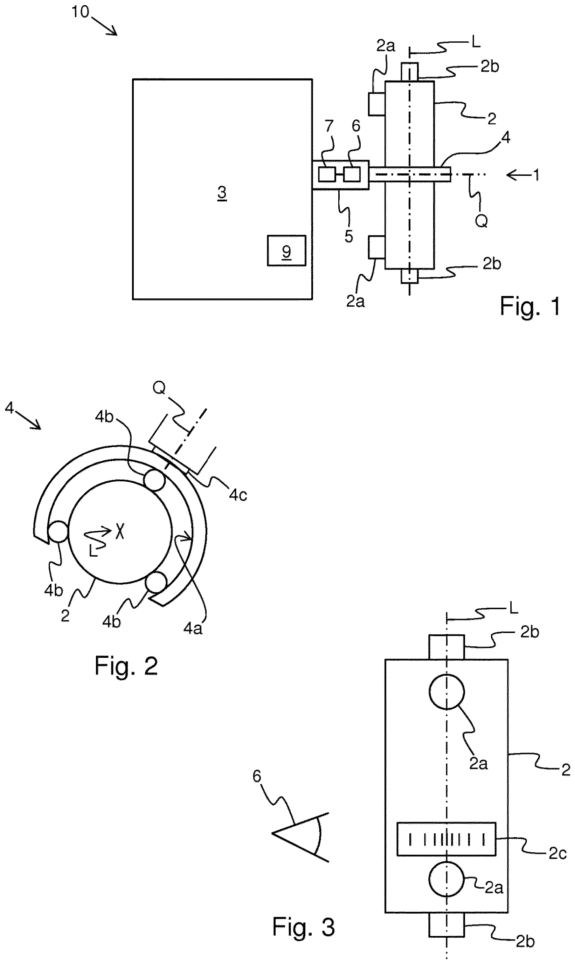

Figur 1 zeigt ein Beispiel eines Systems 10 zur Dialyse in einer schematischen Darstellung mit einem Dialysator 2, einer Dialysevorrichtung 3 und einer Vorrichtung 1 zum Halten des Dialysators 2, die an der Dialysevorrichtung 3 angebracht ist. Die Dialysevorrichtung 3 ist mit dem Dialysator 2 etwa über Schläuche (nicht dargestellt) verbunden und dazu eingerichtet, Blut und Dialysat zur Reinigung des Bluts durch den Dialysator 2 zu leiten.

Der Dialysator 2 ist im vorliegenden Beispiel im Wesentlichen zylinderförmig ausgebildet und weist eine Längsachse L sowie zwei Dialysatkupplungen 2a und zwei Blutkupplungen 2b auf. Über die Blutkupplungen 2b kann die Dialysevorrichtung 3 mittels einer Pumpeinrichtung (nicht dargestellt) durch an die Blutkupplungen 2b angeschlossene Schläuche (nicht dargestellt) Blut eines Patienten in den Dialysator 2 leiten bzw. aus dem Dialysator 2 herausführen. Gleichzeitig kann die Dialysevorrichtung 3 über die Dialysatkupplungen 2a mittels einer weiteren Pumpeinrichtung (nicht dargestellt) durch die an die Dialysatkupplungen 2a angeschlossene Schläuche ein Dialysat zur Reinigung des durch den Dialysator 2 geleiteten Bluts durch den Dialysator 2 leiten. Die Blutkupplungen 2b sind dabei an zwei in Richtung der Längsachse L gegenüberliegenden Enden des Dialysators 2 angeordnet, so dass das Blut im Betrieb des Dialysators 2 im Wesentlichen in Richtung der Längsachse L durch den Dialysator 2 fließt, während die Dialysatkupplungen 2a senkrecht zur Längsachse L auf der gekrümmten Mantelfläche des zylinderförmigen Dialysators 2 angeordnet sind und somit in einer vorgegebenen Drehstellung bezüglich der Längsachse L, wie in Figur 1 dargestellt, der Dialysevorrichtung 3 zugewandt werden können.

Um den Dialysator 2 vor der Inbetriebnahme beispielsweise leicht mit Dialysat und/oder Blut über die entsprechenden Kupplungen 2a, 2b befüllen zu können oder den Dialysator 2 im Betrieb in einer vorteilhaften Betriebsposition, in der ein Benutzer des Systems 100 beispielsweise, wie in Figur 1 dargestellt, nicht durch die Schläuche behindert wird, zu positionieren, ist der Dialysator 2 mittels einer Lagereinrichtung 4 drehbar gelagert. Die Lagereinrichtung 4 kann es insbesondere ermöglichen, den Dialysator 2 in einer Drehstellung bezüglich der Längsachse L und/oder bezüglich einer zur Längsachse L senkrecht verlaufenden Querachse Q auszurichten.

Die Vorrichtung 1 ist zusätzlich mit einer Antriebseinrichtung 5 ausgestattet, die es ermöglicht, den drehbar gelagerten Dialysator 2 automatisch um die Längsachse L und/oder um die Querachse Q zu drehen. Die Antriebseinrichtung kann beispielsweise mindestens einen Schrittmotor aufweisen, um den Dialysator 2 präzise in einer vorgegebenen Drehstellung bezüglich der Längs- und/oder Querachse L, Q, etwa in einer Betriebsposition, zu positionieren.

Um die Ausrichtung des Dialysators 2 zu überwachen und/oder zu steuern, weist die Vorrichtung 1 des Weiteren eine Steuerungseinrichtung 7 auf, die neben der Antriebseinrichtung 5 auch mit einer Sensoreinrichtung 6 zur Erfassung einer Drehung des Dialysators 2 um die Längs- und/oder Querachse L, Q verbunden ist. Anhand der von der Sensoreinrichtung 6 ausgegebenen Sensordaten kann die Steuerungseinrichtung 7 beispielsweise eine gegenwärtige Drehstellung des Dialysators 2 bezüglich der Längs- und/oder Querachse L, Q ermitteln und die Antriebseinrichtung 5 dazu veranlassen, den Dialysator 2 aus der gegenwärtigen Drehstellung in eine gewünschte Betriebsposition zu drehen. Zusätzlich kann die Steuerungseinrichtung 7 auch dazu eingerichtet sein, anhand der Sensordaten eine externe auf den Dialysator 2 einwirkende Kraft zu ermitteln, etwa wenn die Drehung des Dialysators 2 um die Querachse Q in eine Befüllposition, in welcher der Dialysator über die Kupplungen 2a, 2b vor Inbetriebnahme befüllt werden kann, durch einen Schlauch behindert wird, und die Ausrichtung des Dialysators 2 daraufhin zu stoppen, um eine Beschädigung des Dialysators 2, insbesondere der Kupplungen 2a, 2b, der Schläuche und/oder weiterer Komponenten des Systems 10 zu vermeiden.

Die Steuerungseinrichtung 7 kann auch dazu eingerichtet sein, auf Grundlage eines externen Signals, beispielsweise eines von einem Luftblasendetektor 9 der Dialysevorrichtung 3 erzeugten Detektionssignals oder eines von einem Benutzer manuell erzeugten Eingabesignals, mittels der Antriebseinrichtung 5 eine Drehung des Dialysators 2 um die Längs- und/oder Querachse L, Q zu veranlassen. Sollen etwa Luftblasen aus dem Dialysat und/oder Blut entfernt werden oder möchte der Benutzer bei der Befüllung des Dialysators 2 mit Blut und/oder Dialysat eine Luftblasenbildung verhindern, kann die Steuerungseinrichtung 7 die Antriebseinrichtung 5 derart steuern, dass der Dialysator 2 mit hoher Frequenz, etwa mit 1 Hz oder schneller, vorzugsweise mit 5 Hz oder schneller, insbesondere mit 10 Hz oder schneller, um die Längsachse L abwechselnd vor- und zurückgedreht wird und dadurch in Vibration versetzt wird.

Figur 2 zeigt einen Querschnitt eines Beispiels einer Lagereinrichtung 4 einer Vorrichtung (siehe Figur 1) zum Halten eines Dialysators 2. Die schematisch dargestellte Lagereinrichtung 4 ist hierbei als Greifeinrichtung ausgebildet und dazu eingerichtet, den zylinderförmig ausgebildeten Dialysator 2 in einer Ebene senkrecht zu einer Längsachse L des Dialysators 2 zumindest teilweise zu umgreifen. Dabei ist dem Dialysator 2 eine Innenseite 4a der Lagereinrichtung 4 zugewandt, an der drei Rollen 4b angeordnet sind. Die Rollen 4b, welche um jeweils eine parallel zur Längsachse L verlaufende Rollenachse rotieren können, stehen mit der gekrümmten Mantelfläche des Dialysators 2 in Kontakt, so dass der Dialysator 2 um die Längsachse L drehbar gelagert ist.

Die Lagereinrichtung 4 weist zusätzlich ein Gelenk 4c auf, welches eine Drehung zumindest eines Teils der Lagereinrichtung 4, insbesondere des den Dialysator 2 zumindest teilweise umgreifenden Teils der Lagereinrichtung 4, und damit auch des Dialysators 2 um eine senkrecht zur Längsachse L verlaufende Querachse Q erlaubt.

Die Rollen 4b und das Gelenk 4c können mit einer Antriebseinrichtung (nicht dargestellt) verbunden sein, die dazu eingerichtet ist, die Rollen 4b in Rotation zu versetzten und dadurch eine Drehung des Dialysators 2 um die Längsachse L zu bewirken und/oder das Gelenk 4c zu betätigen und dadurch den Dialysator 2 um die Querachse Q zu drehen. Dadurch kann der Dialysator 2 automatisch, insbesondere präzise, komfortabel und zuverlässig, in verschiedene Betriebspositionen verbracht werden, in denen der Dialysator 2 beispielsweise vor Inbetriebnahme leicht mit einer Flüssigkeit, insbesondere Dialysat, Blut und/oder einer Spülflüssigkeit, befüllt werden kann oder im Betrieb einen Benutzer bzw. Patienten nicht behindert.

Figur 3 zeigt eine schematische Darstellung eines Beispiels eines Dialysators 2, der im Wesentlichen zylinderförmig ausgebildet ist und eine Längsachse L, mehrere Kupplungen 2a, 2b zum Anschluss von Schläuchen, über die Blut und/oder Dialysat durch den Dialysator 2 geleitet werden kann, und eine Markierung 2c aufweist.

Die Markierung 2c, im vorliegenden Beispiel ein Strichcode, ist auf der Mantelfläche des Dialysators 2 aufgebracht und verläuft entlang des Umfangs des Dialysators 2, d.h. senkrecht zur Längsachse L. Die Markierung 2c verläuft insbesondere derart auf dem Dialysator 2, dass in einer Drehstellung des Dialysators 2 bezüglich der Längsachse L immer zumindest ein Teil der Markierung 2c durch eine Sensoreinrichtung 6, etwa eine Kamera, erfasst werden kann und entsprechende Sensordaten erzeugt werden können. Dabei ist die Markierung 2c derart ausgebildet, dass aus den entsprechenden Sensordaten eine Information bezüglich der Drehstellung des Dialysators 2 bezüglich der Längsachse L abgeleitet werden kann.

Beispielsweise können Striche des Strichcodes abhängig von ihrer Position relativ zu Dialysatkupplungen 2b des Dialysators 2 unterschiedlich voneinander beabstandet sein. Durch Erfassen von der Sensoreinrichtung 6 zugewandten Strichen des Strichcodes und Ermitteln der Abstände der erfassten Striche zueinander aus den entsprechenden Sensordaten kann somit auf die Position der Dialysatkupplungen 2b relativ zu Sensoreinrichtung 6 und damit gegebenenfalls zu einer Dialysevorrichtung (siehe Figur 1), an welcher die Sensoreinrichtung 6 angeordnet ist, geschlossen werden.

## Patentansprüche

1. Vorrichtung (1) zum Halten eines medizinischen Einmalartikels zur Blutbehandlung mit
- einer Lagereinrichtung (4), die dazu eingerichtet ist, den Einmalartikel drehbar zu lagern,
- einer Antriebseinrichtung (5), die dazu eingerichtet ist, den drehbar gelagerten Einmalartikel um eine Längsachse (L), die im Wesentlichen in einer Richtung verläuft, in der im Betrieb des Einmalartikels Blut durch den Einmalartikel fließt, zu drehen, und
- einer Steuerungseinrichtung (7), die dazu eingerichtet ist, die Antriebseinrichtung (5) zu steuern, **dadurch gekennzeichnet, dass**
- die Vorrichtung (1) ferner eine Sensoreinrichtung (6) aufweist, die dazu eingerichtet ist, eine Drehung des drehbar gelagerten Einmalartikels um wenigstens die Längsachse (L), zu erfassen und entsprechende Sensordaten auszugeben,
- die Steuerungseinrichtung (7) ferner dazu eingerichtet ist, die von der Sensoreinrichtung ausgegebenen Sensordaten zu verarbeiten,
- die Sensoreinrichtung (6) einen optischen Sensor aufweist, der dazu eingerichtet ist, eine am Einmalartikel befindliche Markierung (2c) bei und/oder nach der Drehung des Einmalartikels um die Längsachse (L) zu erfassen und entsprechende erste Sensordaten auszugeben, und
- die Steuerungseinrichtung (7) dazu eingerichtet ist, anhand der vom optischen Sensor ausgegebenen ersten Sensordaten eine Information betreffend die Drehung und/oder die Drehstellung des Einmalartikels bezüglich der Längsachse (L) zu ermitteln.

2. Vorrichtung (1) nach Anspruch 1, wobei
- die Sensoreinrichtung (6) einen Kraftsensor aufweist, der dazu eingerichtet ist, eine auf den Einmalartikel bei der Drehung um wenigstens die Längsachse (L) wirkende Kraft zu erfassen und entsprechende zweite Sensordaten auszugeben, und
- die Steuerungseinrichtung (7) dazu eingerichtet ist, anhand der vom Kraftsensor ausgegebenen zweiten Sensordaten einen Betriebszustand des Einmalartikels zu ermitteln.

3. Vorrichtung (1) nach Anspruch 2, wobei die Steuerungseinrichtung (7) dazu eingerichtet ist, anhand der vom Kraftsensor ausgegebenen zweiten Sensordaten ein Maß für das Gewicht des Einmalartikels zu ermitteln.

4. Vorrichtung (1) nach einem der Ansprüche 2 oder 3, wobei die Steuerungseinrichtung (7) dazu eingerichtet ist, die Antriebseinrichtung (5) anhand der von der Sensoreinrichtung (6) ausgegebenen Sensordaten und/oder des ermittelten Betriebszustands und/oder Maßes für das Gewicht des Einmalartikels zu steuern.

5. Vorrichtung (1) nach einem der vorangehenden Ansprüche, wobei die Steuerungseinrichtung (7) dazu eingerichtet ist, die Antriebseinrichtung (5) derart zu steuern, dass der Einmalartikel abwechselnd vor und zurück um wenigstens die Längsachse (L) gedreht wird.

6. Vorrichtung (1) nach einem der vorangehenden Ansprüche, wobei der medizinische Einmalartikel zur Blutbehandlung als Dialysator (2), Plasmafilter oder Adsorber ausgebildet ist.

7. System (10) zur Blutbehandlung mit
- einer Vorrichtung (1) nach einem der Ansprüche 1 bis 6,
- einem medizinischen Einmalartikel zur Blutbehandlung, der von der Vorrichtung (1) gehalten wird, und
- einer Blutbehandlungsvorrichtung, die mit dem Einmalartikel verbunden und dazu eingerichtet ist, zumindest Blut zur Behandlung des Bluts durch den Einmalartikel zu leiten.

8. System (10) nach Anspruch 7, wobei der Einmalartikel eine Markierung (2c) aufweist, die entlang eines Umfangs des Einmalartikels verläuft.

9. System (10) nach Anspruch 7 oder 8, wobei
- der Einmalartikel wenigstens zwei Öffnungen aufweist, durch die im Betrieb des Einmalartikels eine Flüssigkeit in den Einmalartikel eintreten und/oder aus dem Einmalartikel austreten kann, und
- die Steuerungseinrichtung (7) dazu eingerichtet ist, die Antriebseinrichtung (5) derart zu steuern, dass der Einmalartikel in eine Befüllstellung gedreht wird, in der zumindest eine der Öffnungen im Wesentlichen bis zum vollständigen Befüllen des Einmalartikels mit der Flüssigkeit oberhalb des Flüssigkeitspegels der Flüssigkeit liegt, so dass beim Befüllen des Einmalartikels mit der Flüssigkeit Luft durch die zumindest eine der Öffnungen aus dem Einmalartikel entweichen kann.

10. System (10) nach einem der Ansprüche 7 bis 9, wobei
- die Blutbehandlungsvorrichtung wenigstens einen Detektor (9) aufweist, der dazu eingerichtet ist, in einer durch den Einmalartikel geleiteten Flüssigkeit vor dem Eintritt der Flüssigkeit in den Einmalartikel und/oder nach dem Austritt der Flüssigkeit aus dem Einmalartikel Luftblasen zu detektieren und entsprechende Detektorsignale auszugeben, und
- die Steuerungseinrichtung (7) dazu eingerichtet ist, basierend auf den Detektorsignalen die Antriebseinrichtung (5) derart zu steuern, dass der Einmalartikel abwechselnd vor und zurück um wenigstens die Längsachse (L) gedreht wird.

## Claims

1. An apparatus (1) for holding a disposable medical item for blood treatment, comprising
- a mounting means (4) configured to rotatably mount the disposable item
- a drive means (5) configured to rotate the rotatably mounted disposable item about a longitudinal axis (L) extending substantially in a direction in which blood flows through the disposable item during operation of the disposable item, and
- a control means (7) configured to control the drive means (5), **characterized in that**
- the apparatus (1) further comprises a sensor means (6) configured to detect a rotation of the rotatably mounted disposable item about at least the longitudinal axis (L) and to output corresponding sensor data,
- the control means (7) is further configured to process the sensor data output by the sensor means,
- the sensor means (6) comprises an optical sensor which is configured to detect a mark (2c) located on the disposable item during and/or after the rotation of the disposable item about the longitudinal axis (L) and to output corresponding first sensor data, and
- the control means (7) is configured to determine, on the basis of the first sensor data output by the optical sensor, information relating to the rotation and/or the rotational position of the disposable item with respect to the longitudinal axis (L).

2. The apparatus (1) according to claim 1, wherein
- the sensor means (6) comprises a force sensor which is configured to detect a force acting on the disposable item during rotation about at least the longitudinal axis (L) and to output corresponding second sensor data, and
- the control means (7) is configured to determine an operating state of the disposable item on the basis of the second sensor data output by the force sensor.

3. The apparatus (1) according to claim 2, wherein the control means (7) is configured to determine a measure of the weight of the disposable item on the basis of the second sensor data output by the force sensor.

4. The apparatus (1) according to one of claims 2 or 3, wherein the control means (7) is configured to control the drive means (5) on the basis of the sensor data output by the sensor means (6) and/or the determined operating state and/or measure for the weight of the disposable item.

5. The apparatus (1) according to one of the preceding claims, wherein the control means (7) is configured to control the drive means (5) in such a way that the disposable item is rotated alternately back and forth about at least the longitudinal axis (L)

6. The Apparatus (1) according to one of the preceding claims, wherein the disposable medical item for blood treatment is configured as a dialyzer (2), plasma filter or adsorber.

7. A system (10) for blood treatment comprising
- an apparatus (1) according to any one of claims 1 to 6,
- a disposable medical item for blood treatment held by the apparatus (1), and
- a blood treatment apparatus connected to the disposable item and configured to pass at least blood through the disposable item to treat the blood.

8. The system (10) according to claim 7, wherein the disposable item comprises a marking (2c) extending along a circumference of the disposable item.

9. The system (10) according to claim 7 or 8, wherein
- the disposable item comprises at least two openings through which a liquid can enter the disposable item and/or exit the disposable item during operation of the disposable item, and
- the control means (7) is configured to control the drive means (5) in such a way that the disposable item is rotated into a filling position in which at least one of the openings is substantially above the liquid level of the liquid until the disposable item is completely filled with the liquid, so that air can escape from the disposable item through the at least one of the openings when the disposable item is filled with the liquid.

10. The system (10) according to any one of claims 7 to 9, wherein
- the blood treatment apparatus comprises at least one detector (9) which is configured to detect air bubbles in a liquid passed through the disposable item before the liquid enters the disposable item and/or after the liquid exits the disposable item and to output corresponding detector signals, and
- the control means (7) is configured to control the drive means (5) based on the detector signals in such a way that the disposable item is rotated alternately back and forth about at least the longitudinal axis (L).

## Revendications

1. Dispositif (1) pour maintenir un article à usage unique médical pour le traitement du sang avec
- un système de stockage (4), qui est configuré pour stocker l'article à usage unique de manière rotative,
- un système d'entraînement (5), qui est configuré pour faire tourner l'article à usage unique stocké de manière rotative autour d'un axe longitudinal (L), qui s'étend sensiblement dans une direction dans laquelle lors du fonctionnement de l'article à usage unique le sang circule à travers l'article à usage unique, et
- un système de commande (7), qui est configuré pour commander le système d'entraînement (5), **caractérisé en ce que**
- le dispositif (1) comprend en outre un système de capteur (6), qui est configuré pour détecter une rotation de l'article à usage unique stocké de manière rotative autour d'au moins l'axe longitudinal (L), et pour sortir des données de capteur correspondantes,
- le système de commande (7) est en outre configuré pour traiter les données de capteur sorties par le système de capteur,
- le système de capteur (6) comporte un capteur optique, qui est configuré pour détecter un marquage (2c) se trouvant sur l'article à usage unique lors de et/ou après la rotation de l'article à usage unique autour de l'axe longitudinal (L) et pour sortir des premières données de capteur correspondantes, et
- le système de commande (7) est configuré pour déterminer sur la base des premières données de capteur sorties par le capteur optique une information concernant la rotation et/ou la position rotative de l'article à usage unique par rapport à l'axe longitudinal (L).

2. Dispositif (1) selon la revendication 1, dans lequel
- le système de capteur (6) comporte un capteur de force, qui est configuré pour détecter une force agissant sur l'article à usage unique lors de la rotation autour d'au moins l'axe longitudinal (L) et pour sortir des deuxièmes données de capteur correspondantes, et
- le système de commande (7) est configuré pour déterminer sur la base des deuxièmes données de capteur sorties par le capteur de force un état de fonctionnement de l'article à usage unique.

3. Dispositif (1) selon la revendication 2, dans lequel le système de commande (7) est configuré pour déterminer sur la base des deuxièmes données de capteur sorties par le capteur de force une grandeur pour le poids de l'article à usage unique.

4. Dispositif (1) selon l'une quelconque des revendications 2 ou 3, dans lequel le système de commande (7) est configuré pour commander le système d'entraînement (5) sur la base des données de capteur sorties par le système de capteur (6) et/ou de l'état de fonctionnement déterminé et/ou de la grandeur pour le poids de l'article à usage unique.

5. Dispositif (1) selon l'une quelconque des revendications précédentes, dans lequel le système de commande (7) est configuré pour commander le système d'entraînement (5), de telle sorte que l'article à usage unique est amené en rotation alternativement vers l'avant et vers l'arrière autour d'au moins l'axe longitudinal (L).

6. Dispositif (1) selon l'une quelconque des revendications précédentes, dans lequel l'article à usage unique médical pour le traitement du sang est réalisé sous la forme d'un dialyseur (2), filtre plasma ou adsorbant.

7. Système (10) pour le traitement du sang avec
- un dispositif (1) selon l'une quelconque des revendications 1 à 6,
- un article à usage unique médical pour le traitement du sang, qui est maintenu par le dispositif (1), et
- un dispositif de traitement du sang, qui est relié à l'article à usage unique et configuré pour diriger au moins le sang pour le traitement du sang à travers l'article à usage unique.

8. Système (10) selon la revendication 7, dans lequel l'article à usage unique comporte un marquage (2c), qui s'étend le long d'une périphérie de l'article à usage unique.

9. Système (10) selon la revendication 7 ou 8, dans lequel
- l'article à usage unique comporte au moins deux ouvertures, par lesquelles lors du fonctionnement de l'article à usage unique un liquide peut entrer dans l'article à usage unique et/ou sortir de l'article à usage unique, et
- le système de commande (7) est configuré pour commander le système d'entraînement (5), de telle sorte que l'article à usage unique est amené en rotation dans une position de remplissage, dans laquelle au moins une des ouvertures sensiblement jusqu'au remplissage complet de l'article à usage unique se situe avec le liquide au-dessus du niveau de liquide du liquide, de sorte que lors du remplissage de l'article à usage unique avec le liquide de l'air peut s'échapper de l'article à usage unique par l'au moins une des ouvertures.

10. Système (10) selon l'une quelconque des revendications 7 à 9, dans lequel
- le dispositif de traitement du sang comporte au moins un détecteur (9), qui est configuré pour détecter des bulles d'air dans un liquide guidé à travers l'article à usage unique avant l'entrée du liquide dans l'article à usage unique et/ou après la sortie du liquide de l'article à usage unique et pour émettre des signaux de détecteur correspondants, et
- le système de commande (7) est configuré pour commander le système d'entraînement (5) sur la base des signaux de détecteur, de telle sorte que l'article à usage unique est amené en rotation alternativement vers l'avant et vers l'arrière autour d'au moins l'axe longitudinal (L).
